# EUROPÄISCHE PATENTSCHRIFT

(11) **EP 0 802 789 B1**
(45) Veröffentlichungstag und Bekanntmachung des Hinweises auf die Patenterteilung: **07.07.1999**
(21) Anmeldenummer: 95941722.1
(22) Anmeldetag: 18.12.1995
(51) Int. Cl.: A61K 9/70

(54) **ESTRADIOLHALTIGES PFLASTER**
PLASTER CONTAINING ESTRADIOL
EMPLATRE CONTENANT DE L'ESTRADIOL

(30) Priorität: 12.01.1995 DE 19500662
(43) Veröffentlichungstag der Anmeldung: 29.10.1997
(73) Patentinhaber: LTS LOHMANN Therapie-Systeme GmbH, 56567 Neuwied (DE)
(72) Erfinder: MECONI, Reinhold, D-56567 Neuwied (DE); SEIBERTZ, Frank, D-53557 Bad Hönningen (DE)
(74) Vertreter: Flaccus, Rolf-Dieter, Dr.
(86) Internationale Anmeldenummer: EP9505005
(87) Internationale Veröffentlichungsnummer: WO9621433

(56) Entgegenhaltungen:
- EP-A- 0 305 726
- EP-A- 0 359 625
- DE-A- 4 336 557

## Beschreibung

Die Erfindung betrifft ein wirkstoffhaltiges Pflaster zur kontrollierten Abgabe von Estradiol oder seinen pharmazeutisch unbedenklichen Derivaten alleine oder in Kombination mit Gestagenen an die menschliche oder tierische Haut aus einem Haftkleber aus Ethylcellulose, Estern des Kolophoniums und Laurinsäure, seine Verwendung und ein Verfahren zu seiner Herstellung.

Oestrogenhaltige Pflaster sind bereits bekannt. Sie weisen jedoch die Nachteile auf, daß sie entweder Ethanol enthalten oder die potentielle Gefahr besitzen, daß der Wirkstoff im Laufe der Zeit rekristallisiert.

Aus der DE-OS 32 05 258 und der EP 0 285 563 ist bekannt, Estradiol und Ethanol gleichzeitig in einer Pflasterformulierung zu verabreichen. Die Herstellung dieses Pflasters ist jedoch sehr aufwendig und der Tragekomfort nach Applikation aufgrund der fehlenden Flexibilität gering.

Die EP 0 285 563 beschreibt ein transdermales therapeutisches System für die kombinierte Applikation von Oestrogenen und Gestagenen. Das Reservoir erhält die Wirkstoffformulierung und gegebenenfalls eine Membran sowie Ethanol als perkutanes absorptionsverbesserndes Mittel. Da die Freisetzung des Wirkstoffes hauptsächlich von der Membran gesteuert wird, unterscheidet sich dieses transdermale therapeutische System grundsätzlich von dem Wirkstoffpflaster gemäß der vorliegenden Erfindung. Der Kleber hat bei dem dort beschriebenen Pflaster lediglich die Funktion, das Pflaster auf der Haut zu befestigen. Daß er einen Beitrag zur Steuerung der Wirkstofffreisetzung zu leisten vermag, ist nicht seine Hauptaufgabe, sondern lediglich ein - möglicherweise sogar unerwünschter - Nebeneffekt. Es handelt sich hierbei um ein sogenanntes "Beutelpflaster", da sich die Wirkstoffzubereitung in einem Beutel, bestehend aus undurchlässiger Rückschicht und Membran mit Kleberschicht befindet. Infolge seines komplizierten Aufbaues ist die Herstellung des Pflasters sehr aufwendig, da die Einzelkomponenten separat hergestellt und dann in einem weiteren Arbeitsgang zu einem Pflaster zusammengefügt werden müssen.

Die EP 0 275 716 beschreibt ein - im Gegensatz zu dem erfindungsgemäßen einschichtigen System - zweischichtiges transdermales System zur simultanen Verabreichung von einem oder mehreren Oestrogenen, die in der Polymerschicht gelöst oder mikrodispergiert sind. Die Haftschicht enthält dabei außer den Wirkstoffen Substanzen, die die transdermale Absorption verbessern. Polymer- und Haftschicht können aus Polyacrylaten, Silikonen oder Poliisobutylenen bestehen.

In der EP 0 072 251 ist eine flexible, flüssigkeitsabsorbierende, medikamentöse Bandage beschrieben. Das an der flexiblen Rückschicht befestigte Substrat besteht aus einer hydrophilen Matrix auf der Basis von hydrophilen hochmolekularen Polysacchariden und/oder Polyacrylsäure, Polyacrylamid, Ethylen-Vinylacetat-Copolymeren und anderen Polymeren sowie einer flüssigen Phase auf der Basis einer Lösung oder Emulsion aus Kohlehydrat, Proteinen und mehrwertigen Alkoholen und verschiedenen wirkstoffen, u.a. auch Hormonen. Wesentliches Merkmal dieser Erfindung ist der feuchtigkeitsabsorbierende Kleber.

Die EP 0 328 806 beschreibt ein membranfreies transdermales therapeutisches System, dessen Matrix aus einem Polyacrylatkleber, einem Lösemittel, einem Penetrationsverbesserer und Oestrogen, dessen Derivaten und Kombinationen davon besteht.

In der WO 87/07 138 ist ein Estradiol-Pflaster mit einer Rückschicht, einer den Wirkstoff enthaltenden Matrix und einem Haftkleber, der mit einer wiederablösbaren Schutzschicht abgedeckt ist, beschrieben. Die Herstellung von Matrix und Haftkleber erfolgen in technologisch sehr aufwendigen Arbeitsvorgängen durch Homogenisieren, Entgasen, Beschichten, Trocknen und Vereinzeln. In einer Ausführungsform muß die Rückschicht sogar mit einem Haftkleber beschichtet werden, was einen weiteren Arbeitsgang bedingt. Das Zusammenfügen der einzelnen Teile erfolgt in einem separaten Arbeitsgang. Die Herstellung des Pflasters ist also insgesamt sehr aufwendig und kompliziert.

Aus der US - PS 46 24 665 sind Systeme bekannt, die im Reservoir den Wirkstoff in mikroverkapselter Form enthalten. Das Reservoir ist eingebettet zwischen Rückschicht und einer Membran. Der äußere Rand des Systems ist mit einem Haftkleber ausgerüstet. Der Aufbau und die Herstellung dieses Systems ist sehr kompliziert, da der Wirkstoff mikroverkapselt und in einer flüssigen Phase homogen verteilt werden muß, die dann in weiteren Arbeitsgängen zwischen Rückschicht und Membran eingebettet wird. Zusätzlich muß das System dann mit einem klebenden Rand versehen und mit einer Schutzschicht abgedeckt werden.

Es sind weiterhin aus der EP 0 186 019 Wirkstoffpflaster bekannt, bei denen einer Kautschuk/Klebharzmasse in Wasser quellbare Polymere zugesetzt sind und aus denen Estradiol freigesetzt werden kann. Es hat sich jedoch gezeigt, daß die Freisetzung von Estradiol aus diesen Wirkstoffpflastern viel zu gering ist und nicht den therapeutischen Erfordernissen entspricht.

In der DE-OS 20 06 969 ist ein Pflaster oder ein Haftverband mit Systemwirkung beschrieben, bei dem empfängnisverhütende Substanzen in die Klebstoffkomponente oder den Klebstoffilm eingearbeitet sind. Der Klebstoffilm kann ein Acrylat sein.

Die DE-OS 39 33 460 beschreibt ein oestrogenhaltiges Wirkstoffpflaster auf der Basis von Homo- und/oder Copolymeren mit mindestens einem Derivat der Acryl- oder mit Methacrylsäure in Kombination mit in Wasser quellbaren Substanzen.

Es hat sich jedoch gezeigt, daß haftklebende transdermale therapeutische Matrix-Systeme, die den Wirkstoff teilweise oder vollständig gelöst enthalten, eine zu geringe Wirkstofffreisetzung besitzen und darüberhinaus die potentielle Gefahr in sich bergen, daß der Wirkstoff im Laufe der Zeit rekristallisiert. Dadurch sinkt die Wirkstofffreisetzung und das oestrogenhaltige Pflaster entspricht nicht mehr den therapeutischen Erfordernissen.

Es ist somit Aufgabe der Erfindung, die oben angeführten Nachteile zu vermeiden und ein stabiles, d.h. rekristallisationsfreies, oestrogenhaltiges Pflaster mit ausreichender Wirkstofffreisetzung zur Verfügung zu stellen, dessen Freisetzung sich durch Lagerung nicht verändert.

Überraschend hat sich gezeigt, daß die Aufgabe durch einen oestrogenhaltigen Haftkleber aus Ethylcellulose, Estern des Kolophoniums und Laurinsäure gelöst wird.

Die oben gestellte Aufgabe wird daher durch ein wirkstoffhaltiges Pflaster gemäß Hauptanspruch gelöst. Die Unteransprüche betreffen besonders bevorzugte Ausführungsformen des Erfindungsgegenstandes.

Die Erfindung betrifft somit ein wirkstoffhaltiges Pflaster zur kontrollierten Abgabe von Estradiol oder seinen pharmazeutisch unbedenklichen Derivaten allein oder in Kombination mit Gestagenen, bestehend aus einer Rückschicht, einem damit verbundenen wirkstoffhaltigen Reservoir, das unter Verwendung von Haftklebern hergestellt ist, und einer wiederablösbaren Schutzschicht, wobei der Haftkleber Ethylcellulose, Ester des unhydrierten und/oder hydrierten Kolophoniums und Laurinsäure enthält.

Ethylcellulose ist ein Celluloseether, der durch Reaktion von Ethylchlorid mit Alkalicellulose hergestellt wird. Über die Struktur wird allgemein angenommen, daß ein Cellulosemolekül eine Kette von Anhydroglucose oder Cellobiose-Einheiten ist, die durch Sauerstoffbrücken verbunden sind. Diese langen Ketten von Anhydroglucose mit Sauerstoffbrücken haben eine große Festigkeit bei guter Flexibilität. Diese Eigenschaften werden bei dem erfindungsgemäßen estradiolhaltigen Pflaster ausgenutzt, um dem Haftkleber eine ausreichende Kohäsion zu verleihen, die Voraussetzung für das rückstandsfreie Entfernen des Pflasters von der Haut nach beendeter Applikation ist. Der Haftkleber enthält Ethylcellulose in einem Anteil von 5 - 25 Gew.-%, bevorzugt 8 - 14 Gew.-%.

Zu den Estern des Kolophoniums gehören z.B. der Methylester, der Glycerinester, der Pentaerythritester, der maleinsäuremodifizierte Pentaerythritester, der maleinsäuremodifizierte Glycerinester und der Triethylenglycolester. Der Anteil an Estern des Kolophoniums in dem estrogenhaltigen Haftkleber beträgt 50 - 90 Gew.-%, vorzugsweise 60 - 80 Gew.-%.

Der Haftkleber kann Ester des hydrierten Kolophoniums alleine oder gemeinsam mit Ester des nicht hydrierten Kolophoniums enthalten.

Besonders bevorzugte Ester des Kolophoniums sind der Triethylenglycolester, der Glycerinester und der Pentaerythritester des hydrierten Kolophoniums.

Laurinsäure ist eine basische Carbonsäure mit 12 C-Atomen. Sie bewirkt eine Erhöhung der Penetration von Estradiol durch die Haut. Der Mechanismus ist ungeklärt. Der Haftkleber enthält Laurinsäure in einem Anteil von 1 - 20 Gew.-%, bevorzugt 2 - 15 Gew.-%.

Das rekristallisationsfreie oestrogenhaltige Pflaster mit ausreichender Wirkstofffreisetzung enthält im Reservoir Estradiol und seine pharmazeutisch unbedenklichen Derivate allein oder in Kombination mit Gestagenen in einer Konzentration von insgesamt 1 - 15 Gew.-%, bezogen auf die Gesamtheit der Reservoirbestandteile, und zwar in einem molaren Verhältnis von 1 : 1 bis 1 : 10.

Das estradiolhaltige Reservoir kann mindestens einen Bestandteil der Gruppe enthalten, welche Alterungsschutzmittel, Weichmacher, Antioxidantien und Absorptionsverbesserer umfaßt. Geeignete Weichmacher sind dem Fachmann bekannt und beispielsweise in der DE 37 43 946 beschrieben. Das estradiolhaltige Reservoir enthält üblicherweise Weichmacher in einem Anteil bis zu 5 Gew.-%.

Weiterhin sind im wirkstoffhaltigen Reservoir auch Alterungsschutzmittel in einer Konzentration bis zu 1 Gew.-% enthalten. Diese sind dem Fachman bekannt und z.B. in der DE 37 43 946 beschrieben.

Die Materialien für die undurchlässige Rückschicht und die wiederablösbare Schutzschicht sind dem Fachmann ebenso bekannt (z.B. DE 38 43 239).

Das estradiolhaltige Reservoir kann sowohl aus Lösung als auch aus der Schmelze erzeugt werden.

Ferner kann das Reservoir aus mehreren Schichten bestehen.

Für den Fall, daß das Reservoir keine ausreichende Eigenklebrigkeit zur Haut aufweist, kann dieses mit einer zusätzlichen wirkstofffreien haftklebenden Schicht oder mit einem umlaufenden haftklebenden Rand versehen werden. Auf diese Weise ist gewährleistet, daß das transdermale Pflaster über den gesamten Applikationszeitraum auf der Haut haftet.

Ein besonders bevorzugter Aufbau des transdermalen estradiolhaltigen Pflasters ist ein Matrix-System, bei dem bekanntlich die Matrix die Steuerung für die Wirkstofffreisetzung übernimmt und dem √t-Gesetz nach Higuchi gehorcht. Das bedeutet jedoch nicht, daß nicht in besonderen Fällen auch ein Membran-System von Vorteil ist. Hierbei ist zwischen Reservoir und Haftkleberschicht eine die Wirkstofffreisetzung steuernde Membran angebracht.

Die Dicke des transdermalen Pflasters richtet sich nach den therapeutischen Erfordernissen und kann entsprechend angepaßt werden. Sie liegt üblicherweise im Bereich von 0,03 - 0,6 mm.

### Beispiel 1:

- 75,0 g: Triethylenglycolester von hydriertem Kolophonium (Staybelite Ester 3E/Fa. Hercules) und
- 10,0 g: Glycerinester von hydriertem Kolophonium (Staybelite Ester 10E/Fa. Hercules)
werden bei 100 °C 5 Minuten durch Kneten gemischt. Anschließend werden 2,5 g Estradiol und 2,5 g Laurinsäure zugegeben. Es wird 30 Minuten geknetet. Nach Aufheizen auf 140 °C werden 10,0 g Ethylcellulose N50NF (Fa. Hercules) portionsweise zugegeben und anschließend noch 2,5 St. geknetet.

Die so erhaltene wirkstoffhaltige Klebemasse wird mit einer Hotmelt-Beschichtungsanlage (Düsenauftragssystem) so auf eine wiederablösbare Schutzschicht (Hostaphan RN 100 einseitig mit Silikon beschichtet - Fa. Kalle) beschichtet, daß ein wirkstoffhaltiges Reservoir mit einem Flächengewicht von 80 g/m² resultiert. Auf dieses Reservoir wird die undurchlässige Rückschicht (Polyesterfolie, 15 µm dick) aufkaschiert. Anschließend werden 16 cm² große Wirkstoffpflaster gestanzt.

### Beispiele 2 und 3:

Die Herstellung erfolgt wie unter Beispiel 1 beschrieben, jedoch mit den in Tabelle 1 (Herstellformel) angegebenen Rohstoffen und Mengen.

### Analytik:

- Die Wirkstofffreisetzung der 16 cm²-großen transdermalen Pflaster wird nach der in der USP XXII beschriebenen Ro- tating bottle-Methode in 0,9 %iger Kochsalzlösung bei 37 °C bestimmt.
- Zur Messung der Meerschweinchenpenetration wird die Haut von Meerschweinchen in die Franz-Zelle eingespannt. Auf die Haut wird ein oestradiolhaltiges Pflaster mit einer Fläche von 2,54 cm² aufgeklebt und die Wirkstofffreisetzung bei 37 °C (Akzeptormedium: 0,9 %ige Kochsalzlösung) gemessen (Literatur: Umesh V. Banakar Pharmaceutical dissolution testing (1. Auflage - 1991)).
- Die Prüfung auf Rekristallisationserscheinung wird visuell im Gegenlicht durchgeführt.

Die Ergebnisse sind in Tabelle 2 dargestellt.

**Tabelle 1**

| Zusammensetzung (Angaben in g) | | | | | |
|---|---|---|---|---|---|
| *Bsp.* | *Ethylcellulose N50NF* | *Staybelite Ester* | | *Laurinsäure* | *Estradiol* |
| | | *3E* | *10E* | | |
| 1 | 10,0 | 75,0 | 10,0 | 2,5 | 2,5 |
| 2 | 10,0 | 70,0 | 10,0 | 7,5 | 2,5 |
| 3 | 13,0 | 65,5 | 9,0 | 10,0 | 2,5 |

**Tabelle 2**

| Analysenergebnisse | | | | |
|---|---|---|---|---|
| *Bsp.* | *Estradiolgehalt µg/16cm*^{*2*} | *in vitro-Freisetzung µg/16cm*^{*2*} *· 4 Std.* | *Meerschweinchenhautpenetration µg/16cm*^{*2*} *· 24 Std.* | *Rekristallisation* |
| 1 | 3200 | 645 | 179 | keine |
| 2 | 3200 | 843 | 157 | keine |
| 3 | 3200 | 1368 | 180 | keine |
| nach DE 3933460 | 3200 | 1125 | 95 | erheblich |

Wie die Tabelle zeigt, erhält man eine deutlich bessere Penetration durch die Meerschweinchenhaut gegenüber dem Vergleichsbeispiel nach DE 3933460. Parallel dazu kann festgestellt werden, daß die Rekristallisation bei den erfindungsgemäßen Beispielen völlig unterbleibt.

## Patentansprüche

1. Wirkstoffhaltiges Pflaster zur kontrollierten Abgabe von Estradiol oder seinen pharmazeutisch unbedenklichen Derivaten allein oder in Kombination mit Gestagenen, aus einer Rückschicht, einem damit verbundenen wirkstoffhaltigen Reservoir, das unter Verwendung von Ethylcellulose und Ester des unhydrierten und/oder hydrierten Kolophoniums enthaltenden Haftklebern hergestellt ist, und einer wiederablösbaren Schutzschicht, dadurch gekennzeichnet, daß der Haftkleber ferner Laurinsäure enthält.

2. Wirkstoffhaltiges Pflaster nach Anspruch 1, dadurch gekennzeichnet, daß der Haftkleber Laurinsäure in einem anteil von 1 - 20 Gew.-%, bevorzugt 2 - 15 Gew.-% enthält.

3. Wirkstoffhaltiges Pflaster nach Anspruch 1 oder 2, dadurch gekennzeichnet, daß der Haftkleber 5 - 25 Gew.-% Ethylcellulose, bevorzugt 8 - 14 Gew.-% enthält.

4. Wirkstoffhaltiges Pflaster nach Anspruch 1 oder 2, dadurch gekennzeichnet, daß der Haftkleber Ester des Kolophoniums in einem steil von 50 - 90 Gew.-%, bevorzugt 60 - 80 Gew.-% enthält.

5. Wirkstoffhaltiges Pflaster nach Anspruch 4, dadurch gekennzeichnet, daß der Haftkleber Estradiol oder seine pharmazeutisch unbedenklichen Derivate allein oder in Kombination mit Gestagenen in einem Anteil von 1 - 15 Gew.-%, bevorzugt 1,5 - 5,0 Gew.-% enthält.

6. Wirkstoffhaltiges Pflaster nach einem der Ansprüche 1 - 5, dadurch gekennzeichnet, daß die Rückschicht für die Bestandteile des Reservoirs undurchlässig ist.

7. Wirkstoffhaltiges Pflaster nach einem der Ansprüche 1 - 6, dadurch gekennzeichnet, daß die Ester des Kolophoniums aus der Gruppe, bestehend aus Methylester, Glycerinester, Pentaerythritester, maleinsäuremodifiziertem Penthaerythritester, maleinsäuremodifiziertem Glycerinester und Trietylenglycolester, ausgewählt sind.

8. Wirkstoffhaltiges Pflaster nach einem der Ansprüche 1 - 7, dadurch gekennzeichnet, daß das Reservoir Estradiol oder seine pharmazeutisch unbedenklichen Derivate in Kombination mit Gestagenen in einem molaren Verhältnis von 1 : 1 bis 1 : 10 enthält.

9. Wirkstoffhaltiges Pflaster nach einem der Ansprüche 1 - 8, dadurch gekennzeichnet, daß das Reservoir mindestens einen Bestandteil aus der Gruppe, bestehend aus Alterungsschutzmitteln, Weichmachern, Antioxidantien und Absorptionsverbesserern enthält.

10. Wirkstoffhaltiges Pflaster nach einem der Ansprüche 1 - 9, dadurch gekennzeichnet, daß der Haftkleber ein Lösemittelhaftkleber oder Schmelzhaftkleber ist.

11. Wirkstoffhaltiges Pflaster nach einem der Ansprüche 1 - 10, dadurch gekennzeichnet, daß das Reservoir aus mehreren Schichten besteht und mit einer zusätzlichen wirkstofffreien haftklebenden Schicht versehen sein kann.

12. Wirkstoffhaltiges Pflaster nach einem der Ansprüche 1 - 11, dadurch gekennzeichnet, daß zwischen Reservoir und Haftklebeschicht eine die Wirkstofffreisetzung steuernde Membran angebracht ist.

13. Wirkstoffhaltiges Pflaster nach einem der Ansprüche 1 - 12, dadurch gekennzeichnet, daß das Reservoir mit einem umlaufenden haftklebenden Rand versehen ist.

14. Wirkstoffhaltiges Pflaster nach einem der Ansprüche 1 - 13, dadurch gekennzeichnet, daß die Dicke des wirkstoffhaltigen Pflasters im Bereich von 0,03 - 0,6 mm liegt.

15. Verwendung von Ethylcellulose und Ester von unhydriertem und/oder hydriertem Kolophonium zusammen mit Laurinsäure zur Herstellung eines wirkstoffhaltigen Pflasters nach einem der Ansprüche 1 - 14 für therapeutische Zwecke in der Human- und Veterinärmedizin sowie in der Kosmetik.

## Claims

1. An active substance-containing patch for the controlled release of estradiol or its pharmaceutically acceptable derivatives alone or combined with gestagens, comprising a backing layer, an active substance-containing reservoir which is bonded thereto and produced by using pressure-sensitive adhesives-comprising ethylcellulose and esters of non-hydrogenated and/or hydrogenated colophony, and a removable protective layer, characterized in that the pressure-sensitive adhesive comprises, in addition, lauric acid.

2. The active substance-containing patch according to claim 1 characterized in that the pressure-sensitive adhesive-comprises lauric acid at a proportion of 1 - 20%-wt., preferably 2 - 15%-wt.

3. The active substance-containing patch according to claim 1 or 2 characterized in that the pressure-sensitive adhesive comprises 5 - 25%-wt. of ethylcellulose, preferably 8 - 14%-wt.

4. The active substance-containing patch according to claim 1 or 2 characterized in that the pressure-sensitive adhesive-comprises esters of colophony at a proportion of 50 - 90%-wt., preferably 60 - 80%-wt.

5. The active substance-containing patch according to claim 4 characterized in that the pressure-sensitive adhesive comprises estradiol or its pharmaceutically acceptable derivatives alone or in combination with gestagens at a proportion of 1 - 15%-wt., preferably 1.5 - 5.0%-wt.

6. The active substance-containing patch according to any one of claims 1 - 5 characterized in that the backing layer is impermeable to the components of the reservoir.

7. The active substance-containing patch according to any one of claims 1 - 6 characterized in that the esters of colophony are selected from the group consisting of methyl ester, glycerol ester, pentaerythritol ester, pentaerythritol ester modified with maleic acid, glycerol ester modified with maleic acid, and triethylene glycol ester.

8. The active substance-containing patch according to any one of claims 1 - 7 characterized in that the reservoir comprises estradiol or its pharmaceutically acceptable derivatives in combination with gestagens in a molar ratio of 1 : 1 to 1 : 10.

9. The active substance-containing patch according to anyone of claims 1 -8 characterized in that the reservoir comprises at least one component of the group consisting of anti-ageing agents, plasticizers, antioxidants, and absorption improvers.

10. The active substance-containing patch according to any one of claims 1 - 9 characterized in that the pressure-sensitive adhesive is a solvent-based pressure-sensitive adhesive or a hot-melt pressure-sensitive adhesive.

11. The active substance-containing patch according to any one of claims 1 - 10 characterized in that the reservoir consists of several layers and is provided with an additional pressure-sensitive adhesive layer which is free from active substances.

12. The active substance-containing patch according to any one of claims 1 - 11 characterized in that a membrane which controls the active substance release is located between the reservoir and the pressure-sensitive adhesive layer.

13. The active substance-containing patch according to any one of claims 1 - 12 characterized in that the reservoir is provided with a circumferential pressure-sensitive adhesive edge.

14. The active substance-containing patch according to any one of claims 1 - 13 characterized in that the thickness of the active substance-containing patch is in the range of 0.03 - 0.6 mm.

15. The use of ethyl cellulose and esters of unhydrogenated and/or hydrogenated collophony together with lauric acid for producing an active substance-containing patch according to one of claims 1 - 14 for therapeutic purposes in human and veterinary medicine, as well as in cosmetics.

## Revendications

1. Emplâtre contenant un principe actif pour la libération contrôlée d'oestradiol ou de ses dérivés pharmaceutiquement acceptables, à titre individuel ou en combinaison avec des progestatifs, constitué par une couche dorsale, par un réservoir contenant un principe actif relié à cette dernière, que l'on fabrique en utilisant des agents auto-adhésifs contenant de l'éthylcellulose et des esters de colophane non hydrogénée et/ou hydrogénée, et par une couche de protection pelliculable, caractérisé en ce que l'agent auto-adhésif contient en outre de l'acide laurique.

2. Emplâtre contenant un principe actif selon la revendication 1, caractérisé en ce que l'agent auto-adhésif contient de l'acide laurique en une fraction de 1 à 20% en poids, de préférence de 2 à 15% en poids.

3. Emplâtre contenant un principe actif selon la revendication 1 ou 2, caractérisé en ce que l'agent auto-adhésif contient de l'éthylcellulose à concurrence de 5 à 25% en poids, de préférence de 8 à 14% en poids.

4. Emplâtre contenant un principe actif selon la revendication 1 ou 2, caractérisé en ce que l'agent auto-adhésif contient des esters de colophane en une fraction de 50 à 90% en poids, de préférence de 60 à 80% en poids.

5. Emplâtre contenant un principe actif selon la revendication 4, caractérisé en ce que l'agent auto-adhésif contient de l'oestradiol ou ses dérivés pharmaceutiquement acceptables, à titre individuel ou en combinaison avec des progestatifs, en une fraction de 1 à 15% en poids, de préférence de 1,5 à 5,0% en poids.

6. Emplâtre contenant un principe actif selon l'une quelconque des revendications 1 à 5, caractérisé en ce que la couche dorsale est imperméable pour les constituants du réservoir.

7. Emplâtre contenant un principe actif selon l'une quelconque des revendications 1 à 6, caractérisé en ce que les esters de colophane sont choisis parmi le groupe constitué par l'ester méthylique, l'ester de glycérol, l'ester de pentaérythritol, l'ester de pentaérythritol modifié par de l'acide maléique, l'ester de glycérol modifié par de l'acide maléique et l'ester de triéthylèneglycol.

8. Emplâtre contenant un principe actif selon l'une quelconque des revendications 1 à 7, caractérisé en ce que le réservoir contient de l'oestradiol ou ses dérivés pharmaceutiquement acceptables en combinaison avec des progestatifs dans un rapport molaire de 1 : 1 à 1 : 10.

9. Emplâtre contenant un principe actif selon l'une quelconque des revendications 1 à 8, caractérisé en ce que le réservoir contient au moins un constituant choisi parmi le groupe constitué par des agents de protection contre le vieillissement, par des plastifiants, par des antioxydants et par des agents améliorant l'absorption.

10. Emplâtre contenant un principe actif selon l'une quelconque des revendications 1 à 9, caractérisé en ce que l'agent auto-adhésif est un agent auto-adhésif de type contenant un solvant ou un agent adhésif thermofusible.

11. Emplâtre contenant un principe actif selon l'une quelconque des revendications 1 à 10, caractérisé en ce que le réservoir est constitué par plusieurs couches et peut être muni d'une couche auto-adhésive supplémentaire exempte de principe actif.

12. Emplâtre contenant un principe actif selon l'une quelconque des revendications 1 à 11, caractérisé en ce qu'on applique entre le réservoir et la couche auto-adhésive, une membrane réglant la libération du principe actif.

13. Emplâtre contenant un principe actif selon l'une quelconque des revendications 1 à 12, caractérisé en ce que le réservoir est muni d'un bord périphérique auto-adhésif.

14. Emplâtre contenant un principe actif selon l'une quelconque des revendications 1 à 13, caractérisé en ce que l'épaisseur de l'emplâtre contenant le principe actif se situe dans le domaine de 0,03 à 0,6 mm.

15. Utilisation d'éthylcellulose et d'esters du colophane non hydrogénée et/ou hydrogénée conjointement avec de l'acide laurique pour la préparation d'un emplâtre contenant un principe actif selon l'une quelconque des revendications 1 à 14 à des fins thérapeutiques dans la médecine humaine et vétérinaire, ainsi que dans le domaine cosmétique.
